(19) Europäisches Patentamt
European Patent Office
Office européen des brevets

(11) Veröffentlichungsnummer: **0 105 397**
**B1**

# (12) EUROPÄISCHE PATENTSCHRIFT

(45) Veröffentlichungstag der Patentschrift: **16.07.86**

(51) Int. Cl.[4]: **C 07 D 401/12**, C 07 D 405/14, C 07 D 491/056, A 61 K 31/44

(21) Anmeldenummer: **83109244.0**

(22) Anmeldetag: **19.09.83**

## (54) Schwefelhaltige Indolderivate.

(30) Priorität: **30.09.82 DE 3236243**

(43) Veröffentlichungstag der Anmeldung: **18.04.84 Patentblatt 84/16**

(45) Bekanntmachung des Hinweises auf die Patenterteilung: **16.07.86 Patentblatt 86/29**

(84) Benannte Vertragsstaaten: **AT BE CH DE FR GB IT LI NL SE**

(56) Entgegenhaltungen:
**DE - A - 2 060 816**
**DE - A - 3 034 005**
**US - A - 2 580 475**

**CHEMICAL ABSTRACTS, Vol. 96, No. 15, 12. April 1982, Columbus, Ohio, USA W.J. FANSHAWE et al. "Cis-Mono and disustituted-2-methyl-3-(piperazinyl) and (piperidino)ethyl)indolines and intermediates for their preparation" Seite 685, Spalte 1, abstract no. 122638s**
**CHEMICAL ABSTRACTS, Vol. 97, No. 21, 22. November 1982, Columbus, Ohio, USA D.S. GRIERSON et al. "2-Cyano-delta-piperidines" Seite 858, Spalte 1, abstract no. 182709w**
**CHEMICAL ABSTRACTS, Vol. 92, No. 11, 17. März 1980, Columbus, Ohio, USA K. SUZUKI et al. "Novel alkyl-and phenyl thiomethylation of aromatic compounds" Seite 563, Spalte 1, abstract no. 94100g**

(73) Patentinhaber: **Merck Patent Gesellschaft mit beschränkter Haftung, Frankfurter Strasse 250, D-6100 Darmstadt (DE)**

(72) Erfinder: **Böttcher, Henning, Dr., Soderstrasse 95, D-6100 Darmstadt (DE)**
Erfinder: **Gottschlich, Rudolf, Dr., Buchenweg 1, D-6107 Reinheim (DE)**
Erfinder: **Hausberg, Hans-Heinrich, Dr., Odenwaldstrasse 30, D-6105 Ober-Ramstadt (DE)**
Erfinder: **Seyfried, Christoph, Dr., Mathildenstrasse 6, D-6104 Seeheim-Jugenheim (DE)**
Erfinder: **Minck, Klaus-Otto, Dr., Büchestrasse 8, D-6105 Ober-Ramstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 105 397 B1

ACTORUM AG

**Beschreibung**

Die Erfindung betrifft neue schwefelhaltige Indolderivate der allgemeinen Formel I

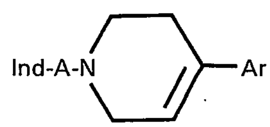

I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

A $-(CH_2)_n-E-C_mH_{2m}-$ oder $-(CH_2)_n-E-C_{m-1}H_{2m-2}CO-$,

n 0 oder 1,

m 2, 3 oder 4,

E S, SO oder $SO_2$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe

bedeuten und

worin

die Alkylgruppen jeweils 1-4 C-Atome besitzen,

sowie deren physiologisch unbedenkliche Säureadditionssalze.

Ähnliche Verbindungen, die antipsychotisch wirken, aber an Stelle der Gruppe A eine Ethylenkette und an Stelle des Indol-3-ylrests einen 2,3-Dihydroindol-3-ylrest enthalten, sind aus der US-PS 4 302 589 bekannt.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, dass die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem dopamin-stimulierende (Anti-Parkinson) Wirkungen. Im einzelnen induzieren die Verbindungen der Formel I contralaterales Drehverhalten in Hemiparkinson-Ratten [feststellbar nach der Methode von Ungerstedt et al., Brain Res. 24, (1970), 485-493] und hemmen die Bindung von tritiierten Dopaminagonisten und -antagonisten an striäre Rezeptoren [feststellbar nach der Methode von Schwarcz et al., J. Neurochemistry, 34, (1980), 772-778 und Creese et al., European J. Pharmacol., 46, (1977), 377-381]. Zusätzlich hemmen die Verbindungen den Zungen-Kieferreflex bei der narkotisierten Ratte [feststellbar in Anlehnung an die Methoden von Barnett et al., European J. Pharmacol. 21, (1973), 178-182, und von Ilhan et al., European J. Pharmacol. 33, (1975), 61-64]. Weiterhin treten analgetische und blutdrucksenkende Wirkungen auf; so wird der bei katheter-tragenden wachen spontan hypertonen Ratten (Stamm SHR/NIH-MO/CHB-EMD; Methode vgl. Weeks und Jones, Proc. Soc. Exptl. Biol. Med. 104, (1960), 646-648] direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die schwefelhaltigen Indolderivate der Formel I sowie ihre physiologisch unbedenklichen Säureadditionssalze.

In den Resten Ind und Ar bedeutet Alkyl vorzugsweise Methyl, ferner auch Ethyl, n-Propyl, Isopropyl, n-Butyl, Isobutyl, sek.-Butyl oder tert.-Butyl. O-Alkyl ist vorzugsweise Methoxy, ferner auch Ethoxy, n-Propoxy, Isopropoxy, n-Butoxy, Isobutoxy, sek.-Butoxy oder tert.-Butoxy. S-Alkyl steht vorzugsweise für Methylthio, aber auch für Ethylthio, n-Propylthio, Isopropylthio, n-Butylthio, Isobutylthio, sek.-Butylthio oder tert.-Butylthio. SO-Alkyl steht vorzugsweise für Methylsulfinyl, ferner auch für Ethylsulfinyl, n-Propylsulfinyl, Isopropylsulfinyl, n-Butylsulfinyl, Isobutylsulfinyl, sek-Butylsulfinyl oder tert.-Butylsulfinyl. $SO_2$-Alkyl ist vorzugsweise Methylsulfonyl, ferner auch Ethylsulfonyl, n-Propylsulfonyl, Isopropylsulfonyl, n-Butylsulfonyl, Isobutylsulfonyl, sek.-Butylsulfonyl oder tert.-Butylsulfonyl.

Der Rest Ind bedeutet insbesondere einen unsubstituierten Indol-3-yl-rest. Falls Ind jedoch einen substituierten Indol-3-ylrest bedeutet, so ist er vorzugsweise einfach, insbesondere in der 2-, 5- oder 6-Stellung substituiert. Weiterhin ist eine Substitution in 1-, 4- oder 7-Stellung möglich. Bevorzugte disubstituierte Indol-3-yl-reste sind in 5,6-Stellung substituiert; Disubstitution ist auch in 1,2-, 1,4-, 1,5-, 1,6-, 1,7-, 2,4-, 2,5-, 2,6-, 2,7-, 4,5-, 4,6-, 4,7-, 5,7- oder 6,7-Stellung möglich. In allen diesen Fällen können die Substituenten gleich oder verschieden sein.

Im einzelnen sind die bevorzugten Substituenten im Benzolring des Restes Ind Methyl, Ethyl Methoxy, Ethoxy, Methylthio, Ethylthio, OH, F, Cl, Br, $CF_3$ und CN. Einige bevorzugte Bedeutungen des Restes Ind sind dementsprechend Indol-3-yl, ferner 1-, 2-, 4-, 5-, 6- oder 7-Methylindol-3-yl, 1-, 2-, 4-, 5-, 6- oder 7-Ethyl-indol-3-yl, 4-, 5-, 6- oder 7-Methoxyindol-3-yl, 4-, 5-, 6- oder 7-Ethoxyindol-3-yl, 4-, 5-, 6- oder 7-Methylthioindol-3-yl, 4-, 5-, 6- oder 7-Ethylthioindol-3-yl, 4-, 5-, 6- oder 7-Methylsulfinylindol-3-yl, 4-, 5-, 6- oder 7-Methylsulfonylindol-3-yl, 4-, 5-, 6- oder 7-Hydroxyindol-3-yl, 4-, 5-, 6- oder 7-Fluorindol-3-yl, 4-, 5-, 6- oder 7-Chlorindol-3-yl, 4-, 5-, 6- oder 7-Bromindol-3-yl, 4-, 5-, 6- oder 7-Trifluormethylindol-3-yl, 4-, 5-, 6- oder 7-Cyanindol-3-yl, 1,2-, 1,4-, 1,5-, 1,6-, 1,7-, 2,4-, 2,5-, 2,6-, 2,7-, 4,5-, 4,6-, 4,7-, 5,6-, 5,7- oder 6,7-Dimethylindol-3-yl, 1-Methyl-4-, -5-, -6- oder -7-methoxyindol-3-yl, 1-Methyl-4-, -5-, -6- oder -7-methylthioindol-3-yl, 1-Methyl-4-, -5-, -6- oder -7-fluorindol-3-yl, 1-Methyl-4-, -5-, -6- oder -7-chlorindol-3-yl, 1-Methyl-4-, -5-, -6- oder -7-bromindol-3-yl, 1-Methyl-4-, -5-, -6- oder -7-trifluormethylindol-3-yl, 1-Methyl-4-, -5-, -6- oder -7-cyanindol-3-yl, 2-Methyl-4-, -5-, -6- oder -7-methoxyindol-3-yl, 2-Methyl-4-, -5-, -6- oder -7-methylthioindol-3-yl, 2-Methyl-4-, -5-, -6- oder -7-fluorindol-3-yl, 2-Methyl-4-, -5-, -6- oder -7-chlorindol-3--yl, 2-Methyl-4-, -5-, -6- oder -7-bromindol-3-yl, 2-Methyl-4-, -5-, -6- oder -7-trifluormethylindol-3-yl, 2-Methyl-4-, -5-, -6- oder -7-cyanindol-3-yl, 4-Methyl-5-fluorindol-3-yl, 5-Fluor-6- oder -7-methylindol-3-yl, 4-Methyl-5-chlorindol-3-yl, 4-Chlor-5-methylindol-3-yl, 5-Methyl-6- oder -7-chlorindol-3-yl, 5-Chlor-6- oder -7-methylindol-3-yl, 4,5-, 4,6-, 4,7-, 5,6-, 5,7- oder 6,7-Dimethoxyindol-3-yl, 4,5-, 4,6-, 4,7-, 5,6-, 5,7- oder 6,7-Dichlorindol-3-yl, 4-Trifluormethyl-5-, -6- oder -7-chlorindol-3-yl.

Der Parameter n ist vorzugsweise 1, der Parameter m ist vorzugsweise 2. E bedeutet vorzugsweise S. Die Gruppe $C_mH_{2m}$ ist vorzugsweise $-(CH_2)_m-$, die Gruppe $C_{m-1}H_{2m-2}$ ist vorzugsweise $-(CH_2)_{m-1}-$ (beide Gruppen sind also vorzugsweise geradkettig).

Dementsprechend ist auch der Rest A vorzugsweise geradkettig; er steht vorzugsweise für $-CH_2-E-CH_2-CH_2-$ oder $-CH_2-E-CH_2-CO-$, im einzelnen vorzugsweise für $-CH_2-S-CH_2CH_2-$, $-CH_2-SO-CH_2CH_2-$, $-CH_2-SO_2-CH_2-CH_2-$, $-CH_2-S-CH_2-CO-$, $-CH_2-SO-CH_2-CO-$, $-CH_2-SO_2-CH_2-CO-$, ferner bevorzugt für $-E-CH_2CH_2-$, $-E-CH_2-CO-$, $-E-(CH_2)_3-$, $-E-CH_2CH_2-CO-$, $-CH_2-E-(CH_2)_3-$, $-CH_2-E-CH_2CH_2-CO-$, $-E-(CH_2)_4-$, $-E-(CH_2)_3-CO-$, $-CH_2-E-(CH_2)_4-$ oder $-CH_2-E-(CH_2)_3-CO-$, im einzelnen bevorzugt für $S-CH_2CH_2-$, $-SO-CH_2CH_2-$, $-SO_2-CH_2CH_2-$, $-S-CH_2-CO-$, $-SO-CH_2-CO-$, $-SO_2-CH_2-CO-$, $-S-(CH_2)_3-$, $-SO-(CH_2)_3-$, $-SO_2-(CH_2)_3-$, $-S-CH_2CH_2-CO-$, $-SO-CH_2CH_2-CO-$, $-SO-CH_2CH_2-CO-$, $-CH_2-S-(CH_2)_3-$, $-CH_2-SO-(CH_2)_3-$, $-CH_2-SO_2-(CH_2)_3-$, $-CH_2-S-CH_2CH_2-CO-$, $-CH_2-SO-CH_2CH_2-CO-$, $-CH_2-SO_2-CH_2CH_2-CO-$, $-S-(CH_2)_4-$, $-SO-(CH_2)_4-$, $-SO_2-(CH_2)_4-$, $-S-(CH_2)_3-CO-$, $-SO-(CH_2)_3-CO-$, $-SO_2-(CH_2)_3-CO-$, $-CH_2-S-(CH_2)_4-$, $-CH_2-SO-(CH_2)_4-$, $-CH_2-SO_2-(CH_2)_4-$, $-CH_2-S-(CH_2)_3-CO-$, $-CH_2-SO-(CH_2)_3-CO-$ oder $-CH_2-SO_2-(CH_2)_3-CO-$; ferner kann der Rest A verzweigt sein und z.B. bevorzugt bedeuten $-E-CH(CH_3)-$, $-CH_2-E-CH(CH_3)-$, $-E-CH(CH_3)-CH_2-$, $-E-CH_2-CH(CH_3)-$, $-E-CH(C_2H_5)-$, $-E-CH(CH_3)-CO-$, $-CH_2-E-CH(CH_3)-CH_2-$, $-CH_2-E-CH_2-CH(CH_3)-$, $-CH_2-E-CH(C_2-H_5)-$, $-CH_2-E-CH(CH_3)-CO-$, $-E-CH(CH_3)-CH_2CH_2-$, $-E-CH_2-CH(CH_3)-CH_2-$, $-E-CH_2CH_2-CH(CH_3)-$, $-E-CH(C_2H_5)-CH_2-$, $-E-CH_2-CH(C_2H_5)-$, $E-CH(C_3H_7)-$, $-E-CH(iso-C_3H_7)-$, $-E-CH(CH_3)-CH(CH_3)-$, $-E-CH(CH_3)-CH_2-CO-$, $-E-CH_2-CH(CH_3)-CO-$, $-E-CH(C_2H_5)-CO-$, $-CH_2-E-CH(CH_3)-CH_2CH_2-$, $-CH_2-E-CH_2-CH(CH_3)-CH_2-$, $-CH_2-E-CH_2CH_2-CH(CH_3)-$, $-CH_2-E-CH(C_2H_5)-CH_2-$, $-CH_2-E-CH_2-CH(C_2H_5)-$, $-CH_2-E-CH(C_3H_7)-$, $-CH_2-E-CH(isoC_3H_7)-$, $-CH_2-E-CH(CH_3)-CH(CH_3)-$, $-CH_2-E-CH(CH_3)-CH_2-CO-$, $-CH_2E-CH_2-CH(CH_3)-CO-$, $-CH_2-E-CH(C_2H_5)-CO-$, im einzelnen bevorzugt $-S-CH(CH_3)-$, $-CH_2-S-CH(CH_3)-$, $-S-CH(CH_3)-CH_2-$, $-S-CH_2-CH(CH_3)-$, $-S-CH(C_2H_5)-$, $S-CH(CH_3)-CO-$, $-CH_2-S-CH(CH_3)-CH_2-$, $-CH_2-S-CH_2-CH(CH_3)-$, $-CH_2-S-CH(C_2H_5)-$, $-CH_2-S-CH(CH_3)-CO-$, $-S-CH(CH_3)-CH_2-CH_2-$, $-S-CH_2-CH(CH_3)-CH_2-$, $-S-CH_2CH_2-CH(CH_3)-$, $-S-CH(C_2H_5)-CH_2-$, $-S-CH_2CH(C_2H_5)-$, $-S-CH(C_3H_7)-$, $-S-CH(iso-C_3H_7)-$, $-S-CH(CH_3)-CH(CH_3)-$, $-S-CH(CH_3)-CH_2-CO-$, $-S-CH_2-CH(CH_3)-CO-$, $-S-CH(C_2H_5)-CO-$, $-CH_2-S-CH(CH_3)-CH_2CH_2-$, $-CH_2-S-CH_2-CH(CH_3)-CH_2-$, $-CH_2-S-CH_2CH_2-CH(CH_3)-$, $-CH_2-S-CH(C_2-H_5)-CH_2-$, $-CH_2-S-CH_2-CH(C_2H_5)-$, $-CH_2-S-CH(C_3H_7)-$, $-CH_2-S-CH(isoC_3H_7)-$, $-CH_2-S-CH(CH_3)-CH(CH_3)-$, $-CH_2-S-CH(CH_3)-CH_2-CO-$, $-CH_2-S-CH_2-CH(CH_3)-CO-$, $-CH_2-S-CH(C_2H_5)-CO-$ sowie die entsprechenden Gruppen, die an Stelle des S-Atoms eine SO-Gruppe oder eine $SO_2$-Gruppe enthalten.

Der Rest Ar ist bevorzugt unsubstituiertes Phenyl. Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind Methyl, F, Cl, Br und Trifluormethyl. Im einzelnen ist Ar bevorzugt Phenyl, o-, m- oder p-Fluorphenyl, o-, m- oder p-Chlorphenyl, o-, m- oder p-Bromphenyl, o-, m- oder p-Tolyl, o-, m- oder p-Trifluormethylphenyl, ferner z.B. o-, m- oder p-Ethylphenyl, o-, m- oder p-n-Propylphenyl, o-, m- oder p-Isopropylphenyl, o-, m- oder p-n-Butylphenyl, o-, m- oder p-Isobutylphenyl, weiterhin Dihalogenphenyl wie 2,3-, 2,4-, 2,5-, 2,6-, 3,4- oder 3,5- Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Fluor-4-chlorphenyl, 2-Brom-4-chlorphenyl; Dimethylphenyl wie 2,3-, 2,4-, 2,5-, 2,6-, 3,4- oder 3,5-Dimethylphenyl; Methyl-chlorphenyl wie 2-Methyl-4-chlorphenyl.

Dementsprechend sind Gegenstand der Erfindung insbesondere diejenigen Verbindungen der Formel I, in denen mindestens einer der genannten Reste eine der vorstehend angegebenen, insbesondere der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste und Parameter die bei Formel I angegebene Bedeutung haben, worin jedoch

- in Ia Ind Indol-3-yl, Methylindol-3-yl, Dimethylindol-3-yl, Methoxyindol-3-yl, Dimethoxyindol-3-yl, Hydroxyindol-3-yl, Dihydroxyindol-3-yl, Fluorindol-3-yl, Chlorindol-3-yl, Dichlorindol-3-yl, Bromindol-3-yl, Cyanindol-3-yl oder Methylendioxyindol-3-yl bedeutet, wobei die Substituenten vorzugsweise in 5- und/oder 6-Stellung stehen;
- in Ib Ind Indol-3-yl, 5- oder 6-Methylindol-3-yl, 5,6-Dimethylindol-3-yl, 5- oder 6-Methoxyindol-3-yl, 5,6-Dimethoxyindol-3-yl, 5- oder 6-Hydroxyindol-3-yl oder 5-Cyanindol-3-yl bedeutet;
- in Ic A $-(CH_2)_n-E-C_mH_{2m}-$ bedeutet;
- in Id m 2 oder 3 bedeutet;
- in Ie E S oder SO bedeutet;
- in If Ar Phenyl, Tolyl, Methoxyphenyl, Fluorphenyl, Chlorphenyl, Trifluormethylphenyl oder Chlortrifluormethylphenyl bedeutet;
- in Ig Ar Phenyl bedeutet;
- in Ih Ind Indol-3-yl, 5-Methoxyindol-3-yl oder 5-Hydroxyindol-3-yl,
  A $-S-CH_2CH_2-$, $-S-CH_2-CO-$, $-S-(CH_2)_3-$, $-S-CH_2CH_2-CO-$, $-CH_2-S-CH_2CH_2-$, $-CH_2-S-CH_2-CO-$, $-CH_2-SO-CH_2CH_2-$, $-CH_2-S-(CH_2)_3-$ oder $-CH_2-S-CH_2CH_2-CO-$ und
  Ar Phenyl, Tolyl, Methoxyphenyl, Fluorphenyl, Chlorphenyl, Trifluormethylphenyl oder Chlor-trifluormethylphenyl
  bedeuten;

in Ii Ind Indol-3-yl,
A $-(CH_2)_n-E-C_mH_{2m}-$,
m 2 oder 3 und
Ar Phenyl
bedeuten;

in Ij Ind Indol-3-yl,
A $-S-CH_2CH_2-$, $-S-(CH_2)_3-$, $-CH_2-S-CH_2CH_2-$, $-CH_2SO-CH_2CH_2-$ oder $-CH_2-S-(CH_2)_3$ und
Ar Phenyl
bedeuten;

in Ik Ind Indol-3-yl,
A $-CH_2-E-CH_2CH_2-$ oder $-CH_2-E-CH_2CO-$ und
Ar Phenyl
bedeuten.

Die Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrere Racemate sowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, dass man eine Verbindung der allgemeinen Formel II

$$Ind-A-X^1 \qquad II$$

worin
$X^1$ X oder $NH_2$ und
X Cl, Br, J, OH oder eine reaktionsfähig funktionell abgewandelte OH-Gruppe bedeuten und
Ind und A die angegebenen Bedeutungen haben,

mit einer Verbindung der allgemeinen Formel III

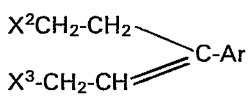
III

worin
$X^2$ und $X^3$ gleich oder verschieden sein können und, falls $X^1 = NH_2$ ist, jeweils X, andernfalls zusammen NH bedeuten und
Ar die angegebene Bedeutung hat
umsetzt

oder dass man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält,

mit einem reduzierenden Mittel behandelt

oder dass man eine sonst der Formel I entsprechende Verbindung, die jedoch an Stelle eines oder mehrerer Wasserstoffatome eine oder mehrere solvolytisch abspaltbare Gruppe(n) enthält,

mit einem solvolysierenden Mittel behandelt

oder dass man eine Verbindung der allgemeinen Formel IV

$$Ind-CH_2N(R)_2 \qquad IV$$

worin
R Alkyl mit 1-4 C-Atomen, beide Reste R zusammen auch $-(CH_2)_p-$ oder $-CH_2CH_2OCH_2CH_2-$ und
p 4 oder 5
bedeuten und
Ind die angegebene Bedeutung hat

mit einem Thiol der allgemeinen Formel V

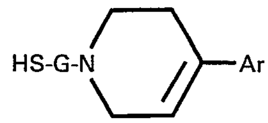
V

worin
G $-C_mH_{2m}-$ oder $-C_{m-1}H_{2m-2}CO-$
bedeutet und
m und Ar die angegebenen Bedeutungen haben
oder einem seiner Salze
umsetzt

oder dass man ein Thiol der allgemeinen Formel VI

$$Ind-SH \qquad VI$$

worin
Ind die angegebene Bedeutung hat
oder eines seiner Salze

mit einer Verbindung der allgemeinen Formel VII

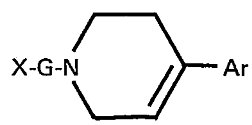
VII

worin X, G und Ar die angegebenen Bedeutungen haben
umsetzt

oder dass man eine Verbindung der Formel VIII

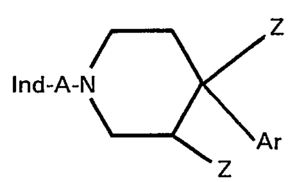
VIII

worin
der eine Rest Z X, CN oder $NH_2$,
der andere Rest Z H bedeutet und
Ind, A, Ar und X die angegebenen Bedeutungen haben
mit einem HZ-abspaltenden Mittel behandelt

und/oder dass man gegebenenfalls in einer Verbindung der Formel I eine CO-Gruppe zu einer $CH_2$-Gruppe reduziert und/oder eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder dass man eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden,

wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II bis VIII können gewünschtenfalls auch in situ gebildet werden, derart, dass man sie aus dem Reaktionsgemisch nicht isoliert, sondern sofort weiter zu den Verbindungen der Formel I umsetzt.

Die Verbindungen der Formel I, insbesondere die Amide I [A = $-(CH_2)_n-E-C_{m-1}H_{2m-2}-CO-$], werden vorzugsweise durch Umsetzung von Indolderivaten der Formel II mit Verbindungen der Formel III erhalten.

In den Indolderivaten der Formel II ist $X^1$ vorzugsweise X; dementsprechend sind in den Verbindungen der Formel III $X^2$ und $X^3$ vorzugsweise zusammen NH. Der Rest C ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Indolderivate der Formel I insbesondere durch Umsetzung der Verbindungen der Formeln Ind-A-Cl oder Ind-A-Br mit Tetrahydropyridinderivaten der Formel III, worin $X^2$ und $X^3$ zusammen eine NH-Gruppe bedeuten (nachstehend als IIIa bezeichnet) erhältlich.

Die Verbindungen der Formel II und insbesondere III sind zum Teil bekannt; die nicht bekannten Verbindungen der Formeln II und III können leicht analog zu den bekannten Verbindungen hergestellt werden. So sind Verbindungen der Formel II (n = 0, E = S) z.B. erhältlich durch Umsetzung von 3-Mercaptoindolen der Formel Ind-SH (VI) mit Halogenverbindungen der Formel Hal-G-$X^1$ (worin Hal Cl, Br oder J bedeutet), z.B. $Cl-CH_2CH_2OH$ oder $Br-CH_2COOH$. Verbindungen der Formel II (n = 1, E = S) können z.B. hergestellt werden aus Mannich-Basen der Formel IV (z.B. Gramin) und Thiolen der Formel HS-G-$X^1$, z.B. $HS-CH_2CH_2OH$ oder $HS-CH_2-COOH$. Die Sulfoxide und Sulfone der Formel II (E = -SO- oder $-SO_2-$) sind durch Oxydation der Thioether (II, E = S) zugänglich.

Primäre Alkohole der Formel II, worin die Gruppe -A-$X^1$ eine $-(CH_2)_n-E-C_{m-1}H_{2m-2}-CH_2OH$-Gruppe bedeutet, z.B. $Ind-CH_2-S-CH_2CH_2OH$, sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-A-Hal, z.B. 3-(4-Chlor-2-thiabutyl)-indol oder -3(4-Brom-2-thiabutyl)-indol. Die entsprechenden Sulfonyloxyverbindungen sind erhältlich aus den Alkoholen Ind-A-OH durch Umsetzung mit den entsprechenden Sulfonsäurechloriden.

Die Jodverbindungen der Formel Ind-A-J, z.B. 3-(4-Jod-2-thiabutyl)-indol, erhält man z.B. durch Einwirkung von Kaliumjodid auf die zugehörigen p-Toluolsulfonsäureester. Die Amine der Formel $Ind-A-NH_2$ sind z.B. aus den Halogeniden mit Phthalimidkalium oder durch Reduktion der entsprechenden Nitrile erhältlich.

Die Piperidinderivate IIIa sind grösstenteils bekannt (vgl. DE-OS 2 060 816) und z.B. erhältlich durch Umsetzung von 4-Piperidon mit metallorganischen Verbindungen der Formel M-AR (worin M ein Li-Atom oder MgHal bedeutet), anschliessende Hydrolyse zu den entsprechenden 4-Ar-4-hydroxy-piperidinen sowie gewünschtenfalls nachfolgende Dehydratisierung zu 4-Ar-3,4-dehydro-piperidinen. Verbindungen der Formel III ($X^2$ und $X^3$ = jeweils X) sind z.B. herstellbar durch Reduktion von 3-Ar-2-penten-1,5-disäurediestern zu 3-Ar-2-penten-1,5-diolen und gegebenenfalls anschliessende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es st aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente $Ind-A-NH_2$ bzw. des Piperidinderivates der Formel IIIa kann günstig sein. Die Herstellung von Säureamiden der Formel I [A = $-(CH_2)_n-E-C_{m-1}H_{2m-2}-CO-$] gelingt z.B. aus den freien Carbonsäuren der Formel $Ind-(CH_2)_n-E-C_{m-1}H_{2m-2}-COOH$ und Tetrahydropyridinen der Formel IIIa in Gegenwart eines Dehydratisierungsmittels, z.B. Carbonyldiimidazol oder Dicyclohexylcarbodiimid in einem der angegebenen inerten Lösungsmittel, bevorzugt THF. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit reduzierenden Mitteln behandelt, vorzugsweise bei Temperaturen zwischen –80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyl, (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen. Vorzugsweise bedient man sich hierzu des nascierenden Wasserstoffs oder komplexer Metallhydride, ferner der Reduktion nach Wolff-Kishner.

Bevorzugte Ausgangsstoffe für die Reduktion entsprechen der Formel IX

Ind'-L-Q-Ar' IX

worin

Ind' einen Indol-3-ylrest, der ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe und/ oder durch eine Arylsulfonylgruppe oder eine Benzylgruppe in 1-Stellung substituiert sein kann,

L eine $-(CH_2)_n-E-C_mH_{2m}$-Kette, worin jedoch eine oder mehrere $-CH_2$-Gruppe(n) durch -CO- und/ oder ein oder mehrere Wasserstoffatome durch OH- Gruppen ersetzt sein können,

Q 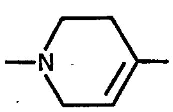 oder 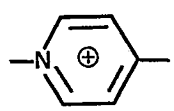 $An^{\ominus}$,

$An^{\ominus}$ ein Anion einer starken Säure und

Ar' eine unsubstituierte oder ein- oder zweifach durch Alkyl, O-Alkyl, S-Alkyl, SO-Alkyl, $SO_2$-Alkyl, OH, F, Cl, Br, $CF_3$, CN und/oder O-Benzyl oder durch eine Methylendioxygruppe substituierte Phenylgruppe bedeuten,

worin jedoch nicht gleichzeitig Ind' = Ind, L = A,

Q 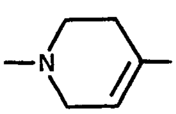 und Ar' = Ar sein können.

In den Verbindungen der Formel VIII ist L bevorzugt $-(CH_2)_n-E-CH_2-CO-CH_2-$, $-(CH_2)_n-E-CH_2-CO-CH_2-CH_2-$ oder $-(CH_2)_n-E-CH_2CH_2-CO-CH_2-$.

Verbindungen der Formel IX sind z.B. herstellbar durch Umsetzung von 4-Ar'-1,2,3,6-tetrahydropyridin oder 4-Ar'-pyridin mit einer Verbindung der Formel X

Ind'-L-$X^1$ X

worin

Ar', Ind', L und $X^1$ die oben angegebenen Bedeutungen haben,

unter den Bedingungen, die oben für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden.

Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmässig eine wässerige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen –80 und + 150°, insbesondere zwischen etwa 0 und 100°.

Besonders vorteilhaft lassen sich -CO-Gruppen in Säureamiden (z.B. solchen der Formel IX worin L eine $-(CH_2)_n-E-C_{m-1}H_{2m-2}$-CO-Gruppe bedeutet) mit $LiAlH_4$ in THF bei Temperaturen zwischen etwa 0 und 66° zu $CH_2$-Gruppen reduzieren. Dabei können in 1-Stellung des Indolrings befindliche Arylsulfonyl-Schutzgruppen gleichzeitig reduktiv abgespalten werden.

Eine Reduktion der Pyridiniumsalze der Formel IX

(worin Q 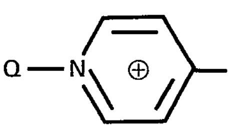 $An^{\ominus}$ und An vorzugsweise Cl oder Br bedeutet) zu Verbindungen der Formel I gelingt z.B. mit $NaBH_4$ in Wasser, Methanol oder Ethanol oder in Gemischen dieser Lösungsmittel, falls erwünscht unter Zusatz einer Base wie NaOH, bei Temperaturen zwischen etwa 0 und 80°.

N-Benzylgruppen können reduktiv mit Natrium in flüssigem Ammoniak abgespalten werden.

Es ist ferner möglich, eine oder mehrere Carbonylgruppen nach der Methode von Wolff-Kishner zu $CH_2$-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxid, umsetzt. Das Reaktionsgemisch wird in der Regel etwa 3-4 Stunden gekocht. Anschliessend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Verbindungen, die sonst der Formel I entsprechen, aber an Stelle eines oder mehrerer H-Atome eine oder mehrere solvolytisch abspaltbare Gruppe(n) enthalten, können zu den Verbindungen der Formel I solvolysiert, insbesondere hydrolysiert werden. Die Ausgangsstoffe für die Solvolyse sind beispielsweise erhältlich durch Reaktion von IIIa mit Verbindungen, die der Formel II ($X^1$ = X) entsprechen, aber an Stelle eines oder mehrerer H-Atome eine oder mehrere solvolytisch abspaltbare Gruppe(n) enthalten. So können 1-Acylindolderivate (entsprechend der Formel I, aber in 1-Stellung des Ind-Restes eine Acylgruppe enthaltend, vorzugsweise eine Alkanoxyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°.

Als basische Katalysatoren verwendet man zweckmässig Natrium-, Kalium- oder Calciumhydroxid, Natrium- oder Kaliumcarbonat, oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Die Indolderivate der Formel I sind ferner durch Umsetzung der Thiole der Formeln V bzw. VI (oder ihrer Salze) mit den Verbindungen der Formeln IV bzw. VII erhältlich.

Die Ausgangsstoffe der Formeln IV bis VII sind teilweise bekannt; die nicht bekannten unter diesen Ausgangsstoffen können leicht analog zu den bekannten Verbindungen hergestellt werden. So sind die Mannich-Basen der Formel IV z.B. aus Indolen der Formel Ind-H, Formaldehyd und Aminen der Formel HN(R )$_2$ erhältlich, die Thiole der Formel V aus den 4-Ar-tetrahydropyridinen der Formel IIIa und Thiolderivaten der Formel HS-G-$X^1$ (wobei die HS-Gruppe auch intermediär geschützt werden kann). In ähnlicher Weise sind die Tetrahydropyridine der Formel VII aus IIIa und Verbindungen der Formel X-G-$X^1$ (z.B. Chloracetylchlorid, 1-Chlor-2-jodethan) zugänglich.

Im einzelnen erfolgt die Umsetzung IV mit V und von VI mit VII in Gegenwart oder Abwesenheit eines inerten Lösungsmittels bei Temperaturen zwischen etwa −20 und 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin; Pyridin oder Picoline; Alkohole wie Methanol, Ethanol oder Butanol; Glykole und Glykolether wie Ethylenglykol, Diethylenglykol, 2-Methoxy-ethanol; Ketone wie Aceton; Ether wie THF oder Dioxan; Amide wie DMF; Sulfoxide wie Dimethylsulfoxid. Auch Gemische dieser Lösungsmittel sind geeignet. Die Thiole der Formeln V und VI werden zweckmässig zunächst in die entsprechenden Mercaptide umgewandelt, vorzugsweise durch Reaktion mit Natrium Kalium, Natrium- oder Kaliumethylat, Natrium- oder Kaliumhydrid in die entsprechenden Natrium- oder Kaliummercaptide.

Man gelangt ferner zu Verbindungen der Formel I, indem man aus Verbindungen der Formel VIII unter Ausbildung einer Doppelbindung HZ abspaltet. Entsprechend der Definition von Z kann es sich z.B. handeln um eine Abspaltung von Halogenwasserstoff, Wasser (Dehydratisierung), einer Carbonsäure oder einer anderen Säure, von Ammoniak oder von HCN. Die Ausganggstoffe der Formel VIII sind z.B. erhältlich durch Umsetzung von II ($X^1$ = X) mit einer Verbindung der Formel XI

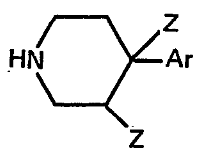

XI

worin Z und Ar die angegebenen Bedeutungen haben.

Falls einer der Reste Z = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, Methanol, Dioxan, THF oder tert.-Butanol. Die als Basen verwendeten Amine können auch im Überschuss als Lösungsmittel eingesetzt werden. Bedeutet der eine der Reste Z eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl- sowie Alkoxysulfonyl-oxy- oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden. In ähnlicher Weise kann HCN aus Verbindungen der Formel VIII (eine Gruppe Z = CN) durch Erhitzen abgespalten werden. Die Eliminierung von HZ aus VIII erfolgt allgemein bei Temperaturen zwischen etwa 0 und etwa 250°, vorzugsweise zwischen 50 und 200°.

Weiterhin kann man gegebenenfalls in einer Verbindung der Formel I eine CO-Gruppe zu einer $CH_2$-Gruppe reduzieren, beispielsweise mit Diboran oder mit einem komplexen Metallhydrid wie $LiAlH_4$ in einem Ether wie THF nach einer der oben angegebenen Methoden.

Weiterhin kann man in einem Thioether der Formel I die Thioethergruppe zu einer SO-Gruppe oder zu einer $SO_2$-Gruppe oder in einem Sulfoxid der Formel I die SO-Gruppe zu einer $SO_2$-Gruppe oxydieren. Die

zu oxydierenden Thioether- oder Sulfoxidgruppen können in der Gruppe A und/oder als Substituent im Rest Ind und/oder im Rest Ar vorhanden sein. Will man die Sulfoxide erhalten, so oxydiert man beispielsweise mit Wasserstoffperoxid, Persäuren wie m-Chlorperbenzoesäure, Cr(VI)-Verbindungen wie Chromsäure, $KMnO_4$, 1-Chlorbenztriazol, Ce(IV)-Verbindungen wie $(NH_4)_2Ce(NO_3)_6$, negativ substituierten aromatischen Diazoniumsalzen wie o- oder p-Nitrophenyldiazoniumchlorid oder elektrolytisch unter verhältnismässig milden Bedingungen und bei relativ niedrigen Temperaturen (etwa −80 bis +100°). Will man dagegen die Sulfone (aus den Thioethern oder den Sulfoxiden) erhalten, so verwendet man die gleichen Oxydationsmittel unter kräftigeren Bedingungen und/oder im Überschuss sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxydationsmittel ist 30%iges wässeriges Wasserstoffperoxid. Dieses führt bei Anwendung der berechneten Menge in Lösungsmitteln wie Essigsäure, Aceton, Methanol, Ethanol oder wässeriger Natronlauge bei Temperaturen zwischen −20 und 100° zu den Sulfoxiden, im Überschuss bei höheren Temperaturen, vorzugsweise in Essigsäure oder in einem Gemisch aus Essigsäure und Acetanhydrid, zu den Sulfonen.

Ether der Formel I, in denen die Reste Ind und/oder Ar ein- oder zweifach durch O-Alkyl substituiert sind, können nach Methoden, die aus der Literatur bekannt sind, gespalten werden, wobei die entsprechenden Hydroxyderivate entstehen. Zum Beispiel kann man die Ether spalten durch Behandeln mit HBr oder HJ in wässeriger oder essigsaurer Lösung, durch Erhitzen mit Lewis-Säuren wie $AlCl_3$ oder Bortrihalogeniden oder durch Verschmelzen mit Pyridin- oder Anilin-hydrohalogeniden, vorzugsweise Pyridinhydrochlorid, bei etwa 150 bis 250°.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malinsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nichtchemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere physiologische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topikale Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline® . Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können verwendet werden bei der therapeutischen Behandlung des menschlichen oder tierischen Körpers und bei der Bekämpfung von Krankheiten, insbesondere von Parkinsonismus, von extrapyramidalen Störungen bei der Neuroleptikatherapie, von Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation und generell als Prolaktin-Hemmer, weiterhin zur Therapie cerebraler Störungen (z.B. Migräne), insbesondere in der Geriatrie ähnlich wie gewisse Ergot-Alkaloide.

Dabei werden die erfindungsgemässen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet «übliche Aufarbeitung»:

Man gibt, falls erforderlich, Wasser hinzu, extrahiert mit einem organischen Lösungsmittel wie Toluol, Chloroform oder Dichlormethan, trennt ab, trocknet die organische Phase über Natriumsulfat, filtriert, dampft ein und reinigt durch Chromatographie und/oder Kristallisation. Temperaturen sind in Celsiusgraden angegeben. Rf-Werte wurden dünnschichtchromatograpisch an Kieselgel erhalten.

*Beispiel 1*

Man rührt eine Lösung von 2,26 g 3-(4-Chlor-2--thiabutyl)-indol [oder 2,70 g 3-(4-Brom-2-thiabutyl)-indol; erhältlich durch Reaktion von Gramin mit 2-Mercaptoethanol zu 3-(4-Hydroxy-2-thiabutyl)-indol und nachfolgende Umsetzung mit $SOCl_2$ oder $PBr_3$] und 1,6 g 4-Phenyl-1,2,3,6-tetrahydropyridin in 10 ml Acetonitril 12 Stunden bei 20°, arbeitet wie üblich auf und erhält 3-[4-(4-Phenyl-1,2,3,6-tetrahydropyridyl)-2-thiabutyl]-indol («P»), F. 109°.

Analog erhält man aus den entsprechenden Chlor- oder Bromthiaalkyl-indolen, z.B.

3-(3-Chlor-1-thiapropyl)-indol [3-(2-Chlorethylthio)--indol]
3-(4-Chlor-1-thiabutyl)-indol
3-(3-Chlor-2-methyl-1-thiapropyl)-indol
3-(4-Chlor-3-methyl-2-thiabutyl)-indol
3-(5-Chlor-1-thiapentyl)-indol
3-(5-Chlor-2-thiapentyl)-indol
3-(6-Chlor-2-thiahexyl)-indol
3-(4-Chlor-2-thiabutyl)-2-methylindol
3-(4-Chlor-2-thiabutyl)-5-methoxyindol
3-(4-Chlor-2-thiabutyl)-6-methoxyindol
3-(4-Chlor-2-thiabutyl)-4-hydroxyindol
3-(4-Chlor-2-thiabutyl)-5-hydroxyindol
3-(4-Chlor-2-thiabutyl)-6-hydroxyindol
3-(4-Chlor-2-thiabutyl)-5-fluorindol
3-(4-Chlor-2-thiabutyl)-5-chlorindol
3-(4-Chlor-2-thiabutyl)-7-bromindol
3-(4-Chlor-2-thiabutyl)-5-cyanindol
3-(4-Chlor-2-thiabutyl)-5,6-methylendioxyindol
3-(4-Chlor-2-thiabutyl)-5,6-dimethylindol
3-(4-Chlor-2-thiabutyl)-5,6-dimethoxyindol
3-(4-Chlor-2-thiabutyl)-5,6-dichlorindol

oder den entsprechenden Sulfoxiden und Sulfonen mit den entsprechenden 4-Aryl-1,2,3,6-tetrahydropyridinen:

3-[3-(4-Phenyl-1,2,3,6-tetrahydropyridyl)-1-thiapropyl]-indol, Hydrochlorid, F. 183-184°
3-[4-(4-Phenyl-1,2,3,6-tetrahydropyridyl)-1-thiabutyl]-indol, Hydrochlorid, F. 191-193°
3-[2-Methyl-3-(4-phenyl-1,2,3,6-tetrahydropyridyl)-1-thiapropyl]-indol, Hydrochlorid, Zers. ab 205°
3-[3-Methyl-4-(4-phenyl-1,2,3,6-tetrahydropyridyl)-2-thiabutyl]-indol, F. 129-131°
3-[5-(4-Phenyl-1,2,3,6-tetrahydropyridyl)-1-thiapentyl]-indol, F. 105-107°
3-[5-(4-Phenyl-1,2,3,6-tetrahydropyridyl)-2-thiapentyl]-indol, F. 132-134°
3-[6-(4-Phenyl-1,2,3,6-tetrahydropyridyl)-2-thiahexyl]-indol
3-[4-(4-Phenyl-1,2,3,6-tetrahydropyridyl)-2-thiabutyl]-2-methylindol
3-[4-(4-Phenyl-1,2,3,6-tetrahydropyridyl)-2-thiabutyl]-5-methoxyindol, Hydrochlorid, F. 182°
3-[4-(4-Phenyl-1,2,3,6-tetrahydropyridyl)-2-thiabutyl]-6-methoxyindol
3-[4-(4-Phenyl-1,2,3,6-tetrahydropyridyl)-2-thiabutyl]-4-hydroxyindol
3-[4-(4-Phenyl-1,2,3,6-tetrahydropyridyl)-2-thiabutyl]-5-hydroxyindol
3-[4-(4-Phenyl-1,2,3,6-tetrahydropyridyl)-2-thiabutyl]-6-hydroxyindol
3-[4-(4-Phenyl-1,2,3,6-tetrahydropyridyl)-2-thiabutyl]-5-fluorindol
3-[4-(4-Phenyl-1,2,3,6-tetrahydropyridyl)-2-thiabutyl]-5-chlorindol
3-[4-(4-Phenyl-1,2,3,6-tetrahydropyridyl)-2-thiabutyl]-7-bromoindol
3-[4-(4-Phenyl-1,2,3,6-tetrahydropyridyl)-2-thiabutyl]-5-cyanindol
3-[4-(4-Phenyl-1,2,3,6-tetrahydropyridyl)-2-thiabutyl]-5,6-methylendioxyindol
3-[4-(4-Phenyl-1,2,3,6-tetrahydropyridyl)-2-thiabutyl]-5,6-dimethylindol
3-[4-(4-Phenyl-1,2,3,6-tetrahydropyridyl)-2-thiabutyl]-5,6-dimethoxyindol, F. 123-124°
3-[4-(4-Phenyl-1,2,3,6-tetrahydropyridyl)-2-thiabutyl]-5,6-dichlorindol
3-[4-(4-o-Tolyl-1,2,3,6-tetrahydropyridyl)-2-thiabutyl]-indol, Hydrochlorid, F. 176-178°
3-[4-(4-m-Tolyl-1,2,3,6-tetrahydropyridyl)-2-thiabutyl]-indol, Hydrochlorid, F. 189-191°
3-[4-(4-p-Tolyl-1,2,3,6-tetrahydropyridyl)-2-thiabutyl]-indol
3-[4-(4-o-Methoxyphenyl-1,2,3,6-tetrahydropyridyl)-2-thiabutyl]-indol
3-[4-(4-m-Methoxyphenyl-1,2,3,6-tetrahydropyridyl)-2-thiabutyl]-indol
3-[4-(4-p-Methoxyphenyl-1,2,3,6-tetrahydropyridyl)-2-thiabutyl]-indol, Hydrochlorid, F. 198°
3-[4-(4-o-Hydroxyphenyl-1,2,3,6-tetrahydropyridyl)-2-thiabutyl]-indol
3-[4-(4-m-Hydroxyphenyl-1,2,3,6-tetrahydropyridyl)-2-thiabutyl]-indol

3-[4-(4-p-Hydroxyphenyl-1,2,3,6-tetrahydropyridyl)-2-thiabutyl]-indol
3-[4-(4-o-Fluorphenyl-1,2,3,6-tetrahydropyridyl)--2-thiabutyl]-indol
3-[4-(4-m-Fluorphenyl-1,2,3,6-tetrahydropyridyl)--2-thiabutyl]-indol
3-[4-(4-p-Fluorphenyl-1,2,3,6-tetrahydropyridyl)--2-thiabutyl]-indol, Hydrochlorid, F. 209°
3-[4-(4-o-Chlorphenyl-1,2,3,6-tetrahydropyridyl)--2-thiabutyl]-indol
3-[4-(4-m-Chlorphenyl-1,2,3,6-tetrahydropyridyl)--2-thiabutyl]-indol
3-[4-(4-p-Chlorphenyl-1,2,3,6-tetrahydropyridyl)--2-thiabutyl]-indol
3-[4-(4-p-Bromphenyl-1,2,3,6-tetrahydropyridyl)--2-thiabutyl]-indol
3-[4-(4-m-Trifluormethylphenyl-1,2,3,6-tetrahydropyridyl)-2-thiabutyl]-indol, Hydrochlorid, F. 166-167°
3-[4-(4-p-Cyanphenyl-1,2,3,6-tetrahydropyridyl)--2-thiabutyl]-indol
3-[4-(4-⟨3,4-Methylendioxyphenyl⟩-1,2,3,6-tetrahydropyridyl)-2-thiabutyl]-indol
3-[4-(4-⟨4-Chlor-3-trifluormethylphenyl⟩-1,2,3,6--tetrahydropyridyl)-2-thiabutyl]-indol, Hydrochlorid, F. 210-211°
3-[4-(4-Phenyl-1,2,3,6-tetrahydropyridyl)-2-thiabutyl]-indol-S-oxid, F. 144-146°
3-[4-(4-Phenyl-1,2,3,6-tetrahydropyridyl)-2-thiabutyl]-indol-S,S-dioxid.

*Beispiel 2*

Ein Gemisch von 4,61 g 3-(4-p-Toluolsulfonyloxy--2-thia-butyl)-indol und 3,18 g 4-Phenyl-1,2,3,6-tetrahydropyridin wird auf 130° erhitzt. Nach Abklingen der exothermen Reaktion und Erkalten arbeitet man wie üblich auf und erhält «P», F. 109°.

Analog erhält man aus den entsprechenden Tosylaten:

3-[4-(4-Phenyl-1,2,3,6-tetrahydropyridyl)-2-thiabutyl]-5-butylindol
3-[4-(4-Phenyl-1,2,3,6-tetrahydropyridyl)-2-thiabutyl]-5-ethoxyindol
3-[4-(4-Phenyl-1,2,3,6-tetrahydropyridyl)-2-thiabutyl]-5-butoxyindol
3-[4-(4-Phenyl-1,2,3,6-tetrahydropyridyl)-2-thiabutyl]-5-methylthioindol
3-[4-(4-Phenyl-1,2,3,6-tetrahydropyridyl)-2-thiabutyl]-5-butylthioindol
3-[4-(4-Phenyl-1,2,3,6-tetrahydropyridyl)-2-thiabutyl]-5-methylsulfinylindol
3-[4-(4-Phenyl-1,2,3,6-tetrahydropyridyl)-2-thiabutyl]-5-methylsulfonylindol.

*Beispiel 3*

Man kocht 3,17 g 3-(4-Jod-2-thiabutyl)-indol, 1,59 g 4-Phenyl-1,2,3,6-tetrahydropyridin und 1,5 g wasserfreies Kaliumcarbonat in 25 ml n-Butanol 2-Stunden unter Rühren, lässt erkalten, arbeitet wie üblich auf und erhält «P», F. 109°.

Analog erhält man mit den entsprechenden 4-Ar--1,2,3,6-tetrahydropyridinen:

3-[4-(4-p-Butoxyphenyl-1,2,3,6-tetrahydropyridyl)--2-thiabutyl]-indol
3-[4-(4-p-Methylthiophenyl-1,2,3,6-tetrahydropyridyl)-2-thiabutyl]-indol
3-[4-(4-p-Butylthiophenyl-1,2,3,6-tetrahydropyridyl)-2-thiabutyl]-indol
3-[4-(4-p-Methylsulfinylphenyl-1,2,3,6-tetrahydropyridyl)-2-thiabutyl]-indol
3-[4-(4-p-Methylsulfonylphenyl-1,2,3,6-tetrahydropyridyl)-2-thiabutyl]-indol.

*Beispiel 4*

Zu einer Lösung von 22,1 g 4-(3-Indolyl)-3-thiabuttersäure (erhältlich aus Gramin und Thioglykolsäure) in 100 ml THF gibt man 16,2 g Carbonyldiimidazol, rührt eine Stunde bei 20° und fügt dann eine Lösung von 15,9 g 4-Phenyl-1,2,3,6-tetrahydropyridin in 50 ml THF zu. Man rührt 16 Stunden bei 20°, arbeitet wie üblich auf und erhält 3-[4-Oxo-4-(4-phenyl-1,2,3,6-tetrahydropyridyl)-2-thiabutyl]-indol, F. 122-125°.

Analog erhält man aus den entsprechenden Carbonsäuren, z.B.

3-(3-Indolyl)-3-thia-proprionsäure (3-Indolylthioessigsäure)
4-(3-Indolyl)-3-thia-buttersäure
3-(3-Indolyl)-2-methyl-3-thiapropionsäure
4-(3-Indolyl)-4-thia-buttersäure
4-(3-Indolyl)-2-methyl-3-thia-buttersäure
5-(3-Indolyl)-4-thia-valeriansäure
5-(3-Indolyl)-5-thia-valeriansäure
6-(3-Indolyl)-5-thia-capronsäure
4-(2-Methylindolyl)-3-thiabuttersäure
4-(5-Methoxyindolyl)-3-thia-buttersäure
4-(6-Methoxyindolyl)-3-thiabuttersäure
4-(4-Hydroxyindolyl)-3-thiabuttersäure
4-(5-Hydroxyindolyl)-3-thiabuttersäure
4-(6-Hydroxyindolyl)-3-thiabuttersäure
4-(5-Fluorindolyl)-3-thiabuttersäure
4-(5-Chlorindolyl)-3-thiabuttersäure
4-(7-Bromindolyl)-3-thiabuttersäure
4-(5-Cyanindolyl)-3-thiabuttersäure
4-(5,6-Methylendioxyindolyl)-3-thiabuttersäure
4-(5,6-Dimethylindolyl)-3-thiabuttersäure
4-(5,6-Dimethoxyindolyl)-3-thiabuttersäure
4-(5,6-Dichlorindolyl)-3-thiabuttersäure

bzw. den entsprechenden Sulfoxiden und Sulfonen mit den entsprechenden 4-Aryl-1,2,3,6-tetrahydropyridinen:

3-[3-Oxo-(4-phenyl-1,2,3,6-tetrahydropyridyl)-1--thiapropyl]-indol, Rf 0,5 (Ethylacetat)
3-[4-Oxo-4-(4-phenyl-1,2,3,6-tetrahydropyridyl)-1--thiabutyl]-indol, Rf 0,5 (Ethylacetat)
3-[2-Methyl-3-oxo-3-(4-phenyl-1,2,3,6-tetrahydropyridyl)-1-thiapropyl]-indol, Rf 0,5 (Ethylacetat)
3-[3-Methyl-4-oxo-(4-phenyl-1,2,3,6-tetrahydropyridyl)-1-thiabutyl]-indol, Rf 0,75 ($CH_2Cl_2/CH_3OH$ 9 : 1)
3-[5-Oxo-5-(4-phenyl-1,2,3,6-tetrahydropyridyl)-1--thiapentyl]-indol, Rf 0,6 (Ethylacetat)
3-[5-Oxo-5-(4-phenyl-1,2,3,6-tetrahydropyridyl)-2--thiapentyl]-indol, F. 133-134°
3-[6-Oxo-6-(4-phenyl-1,2,3,6-tetrahydropyridyl)-2--thiahexyl]-indol
3-[4-Oxo-4-(4-phenyl-1,2,3,6-tetrahydropyridyl)-2--thiabutyl]-2-methylindol

3-[4-Oxo-4-(4-phenyl-1,2,3,6-tetrahydropyridyl)-2--thiabutyl]-5-methoxyindol, Rf 0,78 ($CH_2Cl_2$/Methanol/Ethylacetat 7:2:1)
3-[4-Oxo-4-(4-phenyl-1,2,3,6-tetrahydropyridyl)-2--thiabutyl]-6-methoxyindol
4-[4-Oxo-4-(4-phenyl-1,2,3,6-tetrahydropyridyl)-2--thiabutyl]-4-hydroxyindol
3-[4-Oxo-4-(4-phenyl-1,2,3,6-tetrahydropyridyl)-2--thiabutyl]-5-hydroxyindol
3-[4-Oxo-4-(4-phenyl-1,2,3,6-tetrahydropyridyl)-2--thiabutyl]-6-hydroxyindol
3-[4-Oxo-4-(4-phenyl-1,2,3,6-tetrahydropyridyl)-2--thiabutyl]-5-fluorindol
3-[4-Oxo-4-(4-phenyl-1,2,3,6-tetrahydropyridyl)-2--thiabutyl]-5-chlorindol
3-[4-Oxo-4-(4-phenyl-1,2,3,6-tetrahydropyridyl)-2--thiabutyl]-7-bromindol
3-[4-Oxo-4-(4-phenyl-1,2,3,6-tetrahydropyridyl)-2--thiabutyl]-5-cyanindol
3-[4-Oxo-4-(4-phenyl-1,2,3,6-tetrahydropyridyl)-2--thiabutyl]-5,6-methylendioxyindol
3-[4-Oxo-4-(4-phenyl-1,2,3,6-tetrahydropyridyl)-2--thiabutyl]-5,6-dimethylindol
3-[4-Oxo-4-(4-phenyl-1,2,3,6-tetrahydropyridyl)-2--thiabutyl]-5,6-dimethoxyindol, F. 167-169°
3-[4-Oxo-4-(4-phenyl-1,2,3,6-tetrahydropyridyl)-2--thiabutyl]-5,6-dichlorindol
3-[4-Oxo-4-(4-o-tolyl-1,2,3,6-tetrahydropyridyl)-2--thiabutyl]-indol, F. 118-120°
3-[4-Oxo-4-(4-m-tolyl-1,2,3,6-tetrahydropyridyl)--2-thiabutyl]-indol, F. 98-101°
3-[4-Oxo-4-(4-p-tolyl-1,2,3,6-tetrahydropyridyl)-2--thiabutyl]-indol
3-[4-Oxo-4-(4-o-methoxyphenyl-1,2,3,6-tetrahydropyridyl)-2-thiabutyl]-indol
3-[4-Oxo-4-(4-m-methoxyphenyl-1,2,3,6-tetrahydropyridyl)-2-thiabutyl]-indol
3-[4-Oxo-4-(4-p-methoxyphenyl-1,2,3,6-tetrahydropyridyl)-2-thiabutyl]-indol, F. 115-117°
3-[4-Oxo-4-(4-o-hydroxyphenyl-1,2,3,6-tetrahydropyridyl)-2-thiabutyl]-indol
3-[4-Oxo-4-(4-m-hydroxyphenyl-1,2,3,6-tetrahydropyridyl)-2-thiabutyl]-indol
3-[4-Oxo-4-(4-p-hydroxyphenyl-1,2,3,6-tetrahydropyridyl)-2-thiabutyl]-indol
3-[4-Oxo-4-(4-o-fluorphenyl-1,2,3,6-tetrahydropyridyl)-2-thiabutyl]-indol
3-[4-Oxo-4-(4-m-fluorphenyl-1,2,3,6-tetrahydropyridyl)-2-thiabutyl]-indol
3-[4-Oxo-4-(4-p-fluorphenyl-1,2,3,6-tetrahydropyridyl)-2-thiabutyl]-indol, F. 130-131°
3-[4-Oxo-4-(4-o-chlorphenyl-1,2,3,6-tetrahydropyridyl)-2-thiabutyl]-indol
3-[4-Oxo-4-(4-m-chlorphenyl-1,2,3,6-tetrahydropyridyl)-2-thiabutyl]-indol
3-[4-Oxo-4-(4-p-chlorphenyl-1,2,3,6-tetrahydropyridyl)-2-thiabutyl]-indol
3-[4-Oxo-4-(4-p-bromphenyl-1,2,3,6-tetrahydropyridyl)-2-thiabutyl]-indol
3-[4-Oxo-4-(4-m-trifluormethylphenyl-1,2,3,6-tetrahydropyridyl)-2-thiabutyl]-indol, Rf 0,47 ($CH_2Cl_2$/$CH_3OH$ 95:5)
3-[4-Oxo-4-(4-p-cyanphenyl-1,2,3,6-tetrahydropyridyl)-2-thiabutyl]-indol
3-[4-Oxo-4-(3,4-methylendioxyphenyl-1,2,3,6-tetrahydropyridyl)-2-thiabutyl]-indol
3-[4-Oxo-4-(4-chlor-3-trifluormethylphenyl-1,2,3,6--tetrahydropyridyl)-2-thiabutyl]-indol, F. 124-125°
3-[4-Oxo-4-(4-phenyl-1,2,3,6-tetrahydropyridyl)-2--thiabutyl]-indol-S-oxid
3-[4-Oxo-4-(4-phenyl-1,2,3,6-tetrahydropyridyl)-2--thiabutyl]-indol-S,S-dioxid.

*Beispiel 5*

Ein Gemisch von 2,06 g 3-(4-Amino-2-thiabutyl)--indol [erhältlich durch Reaktion von 3-(4-Brom-2--thiabutyl)-indol mit Phthalimidkalium und anschliessende Hydrolyse] und 2,15 g 1,5-Dichlor-3-phenyl--2-penten (erhältlich durch Reduktion von 3-Phenyl--2-penten-1,5-disäurediethylester mit $LiAlH_4$ und anschliessende Reaktion mit $SOCl_2$) in 40 ml Aceton und 40 ml Wasser wird 24 Stunden gekocht und wie üblich aufgearbeitet. Man erhält «P», F. 109°.

Analog erhält man aus den entsprechenden Aminen und den entsprechenden 1,5-Dichlor-3-Ar-2--pentenen die anderen in den Beispielen 1, 2 und 3 angegebenen Verbindungen der Formel I.

*Beispiel 6*

Zu einer Lösung von 4,25 g 1-[4-(3-Indolyl)-2-thiabutyl]-4-phenylpyridinium-bromid [erhältlich aus 3--(4-Brom-2-thiabutyl)-indol und 4-Phenyl-pyridin] in 50 ml 1n NaOH gibt man unter Rühren 1 g $NaBH_4$ in 20 ml Wasser und rührt danach noch 3 Stunden bei 60°. Nach üblicher Aufarbeitung erhält man «P», F. 109°.

Analog erhält man durch Reduktion der entsprechenden Pyridiniumbromide die anderen in den Beispielen 1, 2 und 3 angegebenen Verbindungen der Formel I.

*Beispiel 7*

Man kocht 4,88 g 1-Benzolsulfonyl-3-[4-(4-Phenyl-1,2,3,6-tetrahydropyridyl)-2-thiabutyl]-indol [erhältlich aus 1-Benzollsulfonyl-3-(4-chlor-2-thiabutyl)-indol und 4-Phenyl-1,2,3,6-tetrahydropyridin] mit 1 g KOH in 7 ml Wasser und 14 ml Ethanol 16 Stunden, konzentriert das Gemisch, arbeitet wie üblich auf und erhält «P», F. 109°.

*Beispiel 8*

Man löst 2,76 g Na in 180 ml Ethanol, gibt 21,9 g 1-(2-Mercaptoethyl)-4-phenyl-1,2,3,6-tetrahydropyridin [erhältlich durch Reaktion von 4-Phenyl--1,2,3,6-tetrahydropyridin mit Thioglykolsäure zu 1-(2-Mercaptoacetyl)-4-phenyl-1,2,3,6-tetrahydropyridin und Reduktion mit $LiAlH_4$] und 17,4 g Gramin hinzu, kocht 16 Stunden, dampft ein, arbeitet wie üblich auf und erhält «P», F. 109°.

*Beispiel 9*

Analog Beispiel 8 erhält man aus 3-Mercaptoindol und 1-(3-Brompropyl)-4-phenyl-1,2,3,6-tetrahydropyridin [erhältlich durch Reaktion von 3-Brompropionsäurebromid mit 4-Phenyl-1,2,3,6-tetrahydropyridin zu 1-(3-Brompropionyl)-4-phenyl-1,2,3,6-tetrahydropyridin und Reduktion mit $LiAlH_4$] das 3-[4--(4-Phenyl-1,2,3,6-tetrahydropyridyl)-1-thiabutyl]--indol, Hydrochlorid, F. 191-193°.

*Beispiel 10*

Man erhitzt 3,80 g 1-Methyl-3-[4-(4-hydroxy-4--phenyl-1-piperidyl)-2-thiabutyl]-indol [erhältlich durch Reaktion von 1-Methyl-3-(4-brom-2-thiabutyl)-indol mit 4-Piperidon, anschliessende Umsetzung mit $C_6H_5Li$ und Hydrolyse] mit 40 ml 1n Salzsäure 2 Stunden auf 50°, arbeitet wie üblich auf und erhält 1-Methyl-3-[4-(4-phenyl-1,2,3,6-tetrahydropyridyl)-2-thiabutyl]-indol.

*Beispiel 11*

Zu einer Suspension von 0,38 g $LiAlH_4$ in 10 ml THF tropft man eine Lösung von 3,62 g 3-[4-Oxo-4--(4-phenyl-1,2,3,6-tetrahydropyridyl)-2-thiabutyl]--indol in 10 ml THF unter Rühren. Nach Abklingen der Reaktion gibt man 5 ml Ethylacetat hinzu, arbeitet wie üblich auf und erhält «P», F. 109°.

Analog erhält man aus den entsprechenden Säureamiden die anderen in Beispiel 1 angegebenen Verbindungen der Formel I.

*Beispiel 12*

Zu einer siedenden Lösung von 3,48 g «P» in 50 ml Ethanol gibt man 6 ml 30%iges $H_2O_2$ und kocht anschliessend 3 Stunden. Nach Zugabe weiterer 4 ml des Oxydationsmittels kocht man noch 9 Stunden, kühlt ab, arbeitet wie üblich auf und erhält 3-[4-(4--Phenyl-1,2,3,6-tetrahydropyridyl)-2-thiabutyl]-indol-S-oxid, F. 144-146°.

Analog erhält man durch Oxydation der entsprechenden Thioether:

3-[3-(4-Phenyl-1,2,3,6-tetrahydropyridyl)-1-thiabutyl]-indol-S-oxid
3-[4-(4-Phenyl-1,2,3,6-tetrahydropyridyl)-1-thiabutyl]-indol-S-oxid
3-[3-Methyl-4-(4-phenyl-1,2,3,6-tetrahydropyridyl)-2-thiabutyl]-indol-S-oxid
3-[5-(4-Phenyl-1,2,3,6-tetrahydropyridyl)-1-thiapentyl]-indol-S-oxid
3-[5-(4-Phenyl-1,2,3,6-tetrahydropyridyl)-2-thiapentyl]-indol-S-oxid
3-[6-(4-Phenyl-1,2,3,6-tetrahydropyridyl)-2-thiahexyl]-indol-S-oxid
3-[4-(4-Phenyl-1,2,3,6-tetrahydropyridyl)-2-thiabutyl]-2-methylindol-S-oxid
3-[4-(4-Phenyl-1,2,3,6-tetrahydropyridyl)-2-thiabutyl]-5-methoxyindol-S-oxid
3-[4-(4-Phenyl-1,2,3,6-tetrahydropyridyl)-2-thiabutyl]-6-methoxyindol-S-oxid
3-[4-(4-Phenyl-1,2,3,6-tetrahydropyridyl)-2-thiabutyl]-4-hydroxyindol-S-oxid
3-[4-(4-Phenyl-1,2,3,6-tetrahydropyridyl)-2-thiabutyl]-5-hydroxyindol-S-oxid
3-[4-(4-Phenyl-1,2,3,6-tetrahydropyridyl)-2-thiabutyl]-6-hydroxyindol-S-oxid
3-[4-(4-Phenyl-1,2,3,6-tetrahydropyridyl)-2-thiabutyl]-5-fluorindol-S-oxid
3-[4-(4-Phenyl-1,2,3,6-tetrahydropyridyl)-2-thiabutyl]-5-chlorindol-S-oxid
3-[4-(4-Phenyl-1,2,3,6-tetrahydropyridyl)-2-thiabutyl]-7-bromindol-S-oxid
3-[4-(4-Phenyl-1,2,3,6-tetrahydropyridyl)-2-thiabutyl]-5-cyanindol-S-oxid
3-[4-(4-Phenyl-1,2,3,6-tetrahydropyridyl)-2-thiabutyl]-5,6-methylendioxyindol-S-oxid
3-[4-(4-Phenyl-1,2,3,6-tetrahydropyridyl)-2-thiabutyl]-5,6-dimethylindol-S-oxid
3-[4-(4-Phenyl-1,2,3,6-tetrahydropyridyl)-2-thiabutyl]-5,6-dimethoxyindol-S-oxid
3-[4-(4-Phenyl-1,2,3,6-tetrahydropyridyl)-2-thiabutyl]-5,6-dichlorindol-S-oxid
3-[4-(4-o-Tolyl-1,2,3,6-tetrahydropyridyl)-2-thiabutyl]-indol-S-oxid
3-[4-(4-m-Tolyl-1,2,3,6-tetrahydropyridyl)-2-thiabutyl]-indol-S-oxid
3-[4-(4-p-Tolyl-1,2,3,6-tetrahydropyridyl)-2-thiabutyl]-indol-S-oxid
3-[4-(4-o-Methoxyphenyl-1,2,3,6-tetrahydropyridyl)-2-thiabutyl]-indol-S-oxid
3-[4-(4-m-Methoxyphenyl-1,2,3,6-tetrahydropyridyl)-2-thiabutyl]-indol-S-oxid
3-[4-(4-p-Methoxyphenyl-1,2,3,6-tetrahydropyridyl)-2-thiabutyl]-indol-S-oxid
3-[4-(4-o-Hydroxyphenyl-1,2,3,6-tetrahydropyridyl)-2-thiabutyl]-indol-S-oxid
3-[4-(4-m-Hydroxyphenyl-1,2,3,6-tetrahydropyridyl)-2-thiabutyl]-indol-S-oxid
3-[4-(4-p-Hydroxyphenyl-1,2,3,6-tetrahydropyridyl)-2-thiabutyl]-indol-S-oxid
3-[4-(4-o-Fluorphenyl-1,2,3,6-tetrahydropyridyl)--2-thiabutyl]-indol-S-oxid
3-[4-(4-m-Fluorphenyl-1,2,3,6-tetrahydropyridyl)--2-thiabutyl]-indol-S-oxid
3-[4-(4-p-Fluorphenyl-1,2,3,6-tetrahydropyridyl)--2-thiabutyl]-indol-S-oxid
3-[4-(4-o-Chlorphenyl-1,2,3,6-tetrahydropyridyl)--2-thiabutyl]-indol-S-oxid
3-[4-(4-m-Chlorphenyl-1,2,3,6-tetrahydropyridyl)--2-thiabutyl]-indol-S-oxid
3-[4-(4-p-Chlorphenyl-1,2,3,6-tetrahydropyridyl)--2-thiabutyl]-indol-S-oxid
3-[4-(4-p-Bromphenyl-1,2,3,6-tetrahydropyridyl)--2-thiabutyl]-indol-S-oxid
3-[4-(4-m-Trifluormethylphenyl-1,2,3,6-tetrahydropyridyl)-2-thiabutyl]-indol-S-oxid
3-[4-(4-p-Cyanphenyl-1,2,3,6-tetrahydropyridyl)--2-thiabutyl]-indol-S-oxid
3-[4-(4-⟨3,4-Methylendioxyphenyl⟩-1,2,3,6-tetrahydropyridyl)-2-thiabutyl]-indol-S-oxid
3-[4-(4-⟨4-Chlor-3-trifluormethylphenyl⟩-1,2,3,6--tetrahydropyridyl)-2-thiabutyl]-indol-S-oxid.

*Beispiel 13*

Zu einer Lösung von 3,48 g «P» in 20 ml Essigsäure gibt man 9 ml 30%iges $H_2O_2$ und kocht 90 Minuten. Nach der üblichen Aufarbeitung erhält man 3-[4-(4--Phenyl-1,2,3,6-tetrahydropyridyl)-2-thiabutyl]-indol-S,S-dioxid.

Analog erhält man durch Oxydation der entsprechenden Thioether:

3-[3-(4-Phenyl-1,2,3,6-tetrahydropyridyl)-1-thiapropyl]-indol-S,S-dioxid
3-[4-(4-Phenyl-1,2,3,6-tetrahydropyridyl)-1-thiabutyl]-indol-S,S-dioxid
3-[3-Methyl-4-(4-phenyl-1,2,3,6-tetrahydropyridyl)-2-thiabutyl]-indol-S,S-dioxid
3-[5-(4-Phenyl-1,2,3,6-tetrahydropyridyl)-1-thiapentyl]-indol-S,S-dioxid
3-[5-(4-Phenyl-1,2,3,6-tetrahydropyridyl)-2-thiapentyl]-indol-S,S-dioxid

3-[6-(4-Phenyl-1,2,3,6-tetrahydropyridyl)-2-thiahexyl]-indol-S,S-dioxid
3-[4-(4-Phenyl-1,2,3,6-tetrahydropyridyl)-2-thiabutyl]-2-methylindol-S,S-dioxid
3-[4-(4-Phenyl-1,2,3,6-tetrahydropyridyl)-2-thiabutyl]-5-methoxyindol-S,S-dioxid
3-[4-(4-Phenyl-1,2,3,6-tetrahydropyridyl)-2-thiabutyl]-6-methoxyindol-S,S-dioxid
3-[4-(4-Phenyl-1,2,3,6-tetrahydropyridyl)-2-thiabutyl]-4-hydroxyindol-S,S-dioxid
3-[4-(4-Phenyl-1,2,3,6-tetrahydropyridyl)-2-thiabutyl]-5-hydroxyindol-S,S-dioxid
3-[4-(4-Phenyl-1,2,3,6-tetrahydropyridyl)-2-thiabutyl]-6-hydroxyindol-S,S-dioxid
3-[4-(4-Phenyl-1,2,3,6-tetrahydropyridyl)-2-thiabutyl]-5-fluorindol-S,S-dioxid
3-[4-(4-Phenyl-1,2,3,6-tetrahydropyridyl)-2-thiabutyl]-5-chlorindol-S,S-dioxid
3-[4-(4-Phenyl-1,2,3,6-tetrahydropyridyl)-2-thiabutyl]-7-bromindol-S,S-dioxid
3-[4-(4-Phenyl-1,2,3,6-tetrahydropyridyl)-2-thiabutyl]-5-cyanindol-S,S-dioxid
3-[4-(4-Phenyl-1,2,3,6-tetrahydropyridyl)-2-thiabutyl]-5,6-methylendioxyindol-S,S-dioxid
3-[4-(4-Phenyl-1,2,3,6-tetrahydropyridyl)-2-thiabutyl]-5,6-dimethylindol-S,S-dioxid
3-[4-(4-Phenyl-1,2,3,6-tetrahydropyridyl)-2-thiabutyl]-5,6-dimethoxyindol-S,S-dioxid
3-[4-(4-Phenyl-1,2,3,6-tetrahydropyridyl)-2-thiabutyl]-5,6-dichlorindol-S,S-dioxid
3-[4-(4-o-Tolyl-1,2,3,6-tetrahydropyridyl)-2-thiabutyl]-indol-S,S-dioxid
3-[4-(4-m-Tolyl-1,2,3,6-tetrahydropyridyl)-2-thiabutyl]-indol-S,S-dioxid
3-[4-(4-p-Tolyl-1,2,3,6-tetrahydropyridyl)-2-thiabutyl]-indol-S,S-dioxid
3-[4-(4-o-Methoxyphenyl-1,2,3,6-tetrahydropyridyl)-2-thiabutyl]-indol-S,S-dioxid
3-[4-(4-m-Methoxyphenyl-1,2,3,6-tetrahydropyridyl)-2-thiabutyl]-indol-S,S-dioxid
3-[4-(4-p-Methoxyphenyl-1,2,3,6-tetrahydropyridyl)-2-thiabutyl]-indol-S,S-dioxid
3-[4-(4-o-Hydroxyphenyl-1,2,3,6-tetrahydropyridyl)-2-thiabutyl]-indol-S,S-dioxid
3-[4-(4-m-Hydroxyphenyl-1,2,3,6-tetrahydropyridyl)-2-thiabutyl]-indol-S,S-dioxid
3-[4-(4-p-Hydroxyphenyl-1,2,3,6-tetrahydropyridyl)-2-thiabutyl]-indol-S,S-dioxid
3-[4-(4-o-Fluorphenyl-1,2,3,6-tetrahydropyridyl)-2-thiabutyl]-indol-S,S-dioxid
3-[4-(4-m-Fluorphenyl-1,2,3,6-tetrahydropyridyl)-2-thiabutyl]-indol-S,S-dioxid
3-[4-(4-p-Fluorphenyl-1,2,3,6-tetrahydropyridyl)-2-thiabutyl]-indol-S,S-dioxid
3-[4-(4-o-Chlorphenyl-1,2,3,6-tetrahydropyridyl)-2-thiabutyl]-indol-S,S-dioxid
3-[4-(4-m-Chlorphenyl-1,2,3,6-tetrahydropyridyl)-2-thiabutyl]-indol-S,S-dioxid
3-[4-(4-p-Chlorphenyl-1,2,3,6-tetrahydropyridyl)-2-thiabutyl]-indol-S,S-dioxid
3-[4-(4-p-Bromphenyl-1,2,3,6-tetrahydropyridyl)-2-thiabutyl]-indol-S,S-dioxid
3-[4-(4-m-Trifluormethylphenyl-1,2,3,6-tetrahydropyridyl)-2-thiabutyl]-indol-S,S-dioxid
3-[4-(4-p-Cyanphenyl-1,2,3,6-tetrahydropyridyl)-2-thiabutyl]-indol-S,S-dioxid
3-[4-(4-⟨3,4-Methylendioxyphenyl⟩-1,2,3,6-tetrahydropyridyl)-2-thiabutyl]-indol-S,S-dioxid
3-[4-(4-⟨4-Chlor-3-trifluormethylphenyl⟩-1,2,3,6-tetrahydropyridyl)-2-thiabutyl]-indol-S,S-dioxid.

*Beispiel 14*

Ein Gemisch aus 4,25 g 3-[4-(4-p-Methoxyphenyl-1,2,3,6-tetrahydropyridyl)-2-thiabutyl]-indol-hydrochlorid und 3,5 g Pyridinhydrochlorid wird 3 Stunden bei 160° gerührt. Nach üblicher Aufarbeitung erhält man 3-[4-(4-p-Hydroxyphenyl-1,2,3,6-tetrahydropyridyl)-2-thiabutyl]-indol.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 3-[4-(4-Phenyl-1,2,3,6-tetrahydropyridyl)-2-thiabutyl]-indol, 4 kg Lactose, 1,2 kg Kartoffelstärke, 0,2 kg Talk und 0,1 kg Magnesiumstearat wird in üblicher Weise zu Tabletten verpesst, derart, dass jede Tablette 10 mg Wirkstoff enthält.

Beispiel B: Dragees

Analog Beispiel A werden Tabletten gepresst, die anschliessend in üblicher Weise mit einem Überzug aus Saccharose, Kartoffelstärke, Talk, Tragant und Farbstoff überzogen werden.

Beispiel C: Kapseln

2 kg 3-[4-(4-Phenyl-1,2,3,6-tetrahydropyridyl)-2-thiabutyl]-indol werden in üblicher Weise in Hartgelatinekapseln gefüllt, so dass jede Kapsel 20 mg des Wirkstoffs enthält.

Beispiel D: Ampullen

Eine Lösung von 1 kg 3-[4-(4-Phenyl-1,2,3,6-tetrahydropyridyl)-1-thiabutyl]-indol-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihrer physiologisch unbedenklichen Säureadditionssalze enthalten.

**Patentansprüche**

1. Schwefelhaltige Indolderivate der allgemeinen Formel I

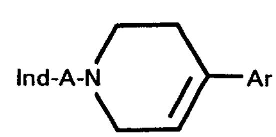 I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch

eine Methylendioxygruppe substituiert sein kann,

A $-(CH_2)_n-E-C_mH_{2m}-$ oder $-(CH_2)_n-E-C_{m-1}H_{2m-2}CO-$,

n 0 oder 1,

m 2, 3 oder 4,

E S, SO oder $SO_2$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe

bedeuten und

worin

die Alkylgruppen jeweils 1-4 C-Atome besitzen, sowie deren physiologisch unbedenkliche Säureadditionssalze.

2. 3-[4-(4-Phenyl-1,2,3,6-tetrahydropyridyl)-2--thiabutyl]-indol.

3. Verfahren zur Herstellung von schwefelhaltigen Indolderivaten der allgemeinen Formel I

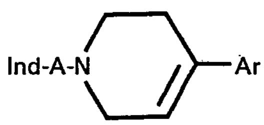

I

worin

Ind einen Indol-3-ylrest, der ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituiert sein kann,

A $-(CH_2)_n-E-C_mH_{2m}-$ oder $-(CH_2)_n-E-C_{m-1}H_{2m-2}CO-$,

n 0 oder 1,

m 2, 3 oder 4,

E S, SO oder $SO_2$ und

Ar eine unsubstituierte oder ein- oder zweifach durch Alkyl, O-Alkyl, S-Alkyl, SO-Alkyl, $SO_2$-Alkyl, OH, F, Cl, Br, $CF_3$ und/oder CN oder durch eine Methylendioxygruppe substituierte Phenylgruppe

bedeuten und

worin

die Alkylgruppen jeweils 1-4 C-Atome besitzen, sowie von deren physiologisch unbedenklichen Säureadditionssalzen, dadurch gekennzeichnet, dass man eine Verbindung der allgemeinen Formel II

$$\text{Ind-A-X}^1 \qquad \text{II}$$

worin

$X^1$ X oder $NH_2$ und

X Cl, Br, J, OH oder eine reaktionsfähig funktionell abgewandelte OH-Gruppe bedeuten und

Ind und A die angegebenen Bedeutungen haben, mit einer Verbindung der allgemeinen Formel III

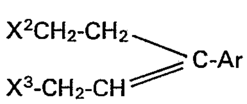

III

worin

$X^2$ und $X^3$ gleich oder verschieden sein können und, falls $X^1 = NH_2$ ist, jeweils X, andernfalls zusammen NH bedeuten und

Ar die angegebene Bedeutung hat

umsetzt

oder dass man eine sonst der Formel I entsprechende Verbindung, die jedoch an Stelle eines oder mehrerer Wasserstoffatome eine oder mehrere reduzierbare Gruppe(n) und/oder eine oder mehrere zusätzliche C-C- und/oder C-N-Bindung(en) enthält,

mit einem reduzierenden Mittel behandelt

oder dass man eine sonst der Formel I entsprechende Verbindung, die jedoch an Stelle eines oder mehrerer Wasserstoffatome eine oder mehrere solvolytisch abspaltbare Gruppe(n) enthält,

mit einem solvolysierenden Mittel behandelt

oder dass man eine Verbindung der allgemeinen Formel IV

$$\text{Ind-CH}_2\text{N(R)}_2 \qquad \text{IV}$$

worin

R Alkyl mit 1-4 C-Atomen, beide Reste R zusammen auch $-(CH_2)_p-$ oder $-CH_2CH_2OCH_2CH_2-$ und

p 4 oder 5

bedeuten und

Ind die angegebene Bedeutung hat

mit einem Thiol der allgemeinen Formel V

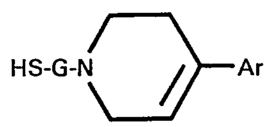

V

worin

G $-C_mH_{2m}-$ oder $-C_{m-1}H_{2m-2}CO-$

bedeutet und

m und Ar die angegebenen Bedeutungen haben

oder einem seiner Salze

umsetzt

oder dass man ein Thiol der allgemeinen Formel VI

$$\text{Ind-SH} \qquad \text{VI}$$

worin

Ind die angegebene Bedeutung hat

oder eines seiner Salze

mit einer Verbindung der allgemeinen Formel VII

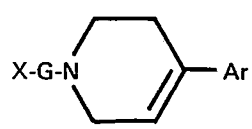

VII

worin X, G und Ar die angegebenen Bedeutungen haben

umsetzt

oder dass man eine Verbindung der Formel VIII

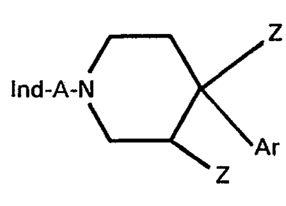

VIII

worin
der eine Rest Z X, CN oder $NH_2$,
der andere Rest Z H bedeutet und
Ind, A, Ar und X die angegebenen Bedeutungen haben
mit einem HZ-abspaltenden Mittel behandelt und/oder dass man gegebenenfalls in einer Verbindung der Formel I eine CO-Gruppe zu einer $CH_2$-Gruppe reduziert und/oder eine Thioethergruppe zu einer SO-Gruppe oder $SO_2$-Gruppe oder eine SO-Gruppe zu einer $SO_2$-Gruppe oxydiert und/oder eine Alkoxygruppe unter Bildung einer OH-Gruppe spaltet und/oder dass man eine erhaltene Base der Formel I durch Behandeln mit einer Säure in eines ihrer physiologisch unbedenklichen Säureadditionssalze umwandelt.

4. Verfahren zur Herstellung pharmazeutischer Zubereitungen, dadurch gekennzeichnet, dass man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, gekennzeichnet durch einen Gehalt an mindestens einer Verbindung der allgemeinen Formel I und/oder einem ihrer physiologisch unbedenklichen Säureadditionssalze.

6. Verbindungen der allgemeinen Formel I nach Patentanspruch 1 zur Bekämpfung von Krankheiten.

7. Verwendung von Verbindungen der allgemeinen Formel I und/oder ihrer physiologisch unbedenklichen Säureadditionssalze zur Herstellung von Arzneimitteln.

**Claims**

1. Sulfur-containing indole derivatives of the general formula I

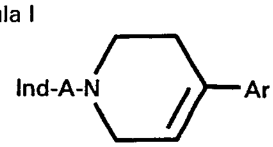 I

wherein Ind is a 3-indolyl radical which can be substituted once or twice by alkyl, O-alkyl, S-alkyl, SO-alkyl, $SO_2$-alkyl, OH, F, Cl, Br, $CF_3$ and/or CN or by a methylenedioxy group, A is $-(CH_2)_n-E-C_mH_{2m}-$ or $-(CH_2)_n-E-C_{m-1}H_{2m-2}CO-$, n is 0 or 1, m is 2, 3 or 4, E is S, SO or $SO_2$ and Ar is a phenyl group which is unsubstituted or substituted once or twice by alkyl, O-alkyl, S-alkyl, SO-alkyl, $SO_2$-alkyl, OH, F, Cl, Br, $CF_3$ and/or CN or by a methylenedioxy group and wherein the alkyl groups each have 1-4 C atoms, and their physioligically acceptable acid addition salts.

2. 3-[4-(4-Phenyl-1,2,3,6-tetrahydropyridyl)-2--thiabutyl]indole.

3. Process for the preparation of sulfur-containing indole derivatives of the general formula I

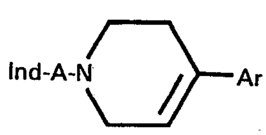 I

wherein Ind is a 3-indolyl radical which can be substituted once or twice by alkyl, O-alkyl, S-alkyl, SO-alkyl, $SO_2$-alkyl, OH, F, Cl, Br, $CF_3$ and/or CN or by a methylenedioxy group, A is $-(CH_2)_n-E-C_mH_{2m}-$ or $-(CH_2)_n-E-C_{m-1}H_{2m-2}CO-$, n is 0 or 1, m is 2, 3 or 4, E is S, SO or $SO_2$ and Ar is a phenyl group which is unsubstituted or substituted once or twice by alkyl, O-alkyl, S-alkyl, SO-alkyl, $SO_2$-alkyl, OH, F, Cl, Br, $CF_3$ and/or CN or by a methylenedioxy group and wherein the alkyl groups each have 1-4 C atoms, and their physioligically acceptable acid addition salts, characterised in that a compound of the general formula II

$$Ind-A-X^1 \qquad II$$

wherein $X^1$ is X or $NH_2$ and X is Cl, Br, I, OH or a reactive functionally modified OH group and Ind and A have the indicated meanings, is reacted with a compound of the general formula III

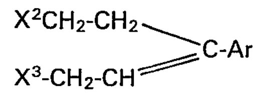 III

wherein $X^2$ and $X^3$ can be identical or different and, if $X^1$ is $NH_2$, are each X but otherwise are together NH, and Ar has the indicated meaning; or a compound which otherwise corresponds to the formula I but contains, in place of one or more hydrogen atoms, one or more reducible group(s) and/or one or more additional C-C and/or C-N bond(s) is treated with a reducing agent; or a compound which otherwise corresponds to the formula I but contains, in place of one or more hydrogen atoms, one or more group(s) which can be split off solvolytically, is treated with a solvolysing agent; or a compound of the general formula IV

$$Ind-CH_2N(R)_2 \qquad IV$$

wherein R is alkyl having 1-4 C atoms, or both radicals R together are also $-(CH_2)_p-$ or $-CH_2CH_2OCH_2CH_2-$ and p is 4 or 5 and Ind has the indicated meaning is reacted with a thiol of the general formula V

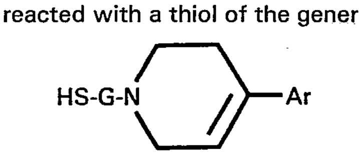 V

wherein G is $-C_mH_{2m}-$ or $-C_{m-1}H_{2m-2}CO-$ and m and Ar have the indicated meanings, or one of its salts; or a thiol of the general formula VI

$$Ind-SH \qquad VI$$

wherein Ind has the indicated meaning, or one of its salts, is reacted with a compound of the general formula VII

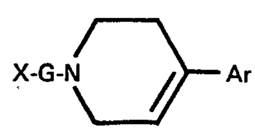 VII

wherein X, G and Ar have the indicated meanings; or a compound of the formula VIII

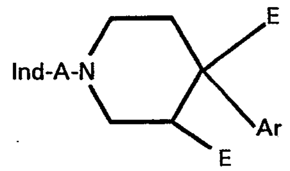

VIII

wherein one radical E is X, CN or $NH_2$ and the other radical E is H and Ind, A, Ar and X have the indicated meanings is treated with an agent which splits off HE; and/or, where appropriate, in a compound of the formula I, a CO group is reduced to give a $CH_2$ group and/or a thioether group is oxidised to give a SO group or $SO_2$ group, or a SO group is oxidised to give a $SO_2$ group and/or an alkoxy group is cleaved with the formation of an OH group and/or a base of the formula I obtained is converted into one of its physiologically acceptable acid addition salts by treatment with an acid.

4. Process for the preparation of pharmaceutical formulations, characterised in that a compound of the formula I and/or one of its physiologically acceptable acid addition salts is converted into a suitable dosage form together with at least one solid, liquid or semiliquid vehicle or auxiliary and optionally in combination with one or more other active compounds.

5. Pharmaceutical formulation characterised by a content of at least one compound of the general formula I and/or one of its physiologically acceptable acid addition salts.

6. Compounds of the general formula I according to Patent Claim 1 for controlling diseases.

7. Use of compounds of the general formula I and/or their physiologically acceptable acid addition salts for the preparation of medicaments.

**Revendications**

1. Dérivés d'indole contenant du soufre de formule générale I

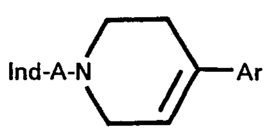

I

où

Ind représente un radical indol-3-yle, qui peut être mono- ou disubstitué par alcoyle, O-alcoyle, S-alcoyle, SO-alcoyle, $SO_2$-alcoyle, OH, F, Cl, Br, $CF_3$ et/ou CN par un groupe méthylènedioxy,

A représente $-(CH_2)_n-E-C_mH_{2m}-$ ou $-(CH_2)_n-E-C_{m-1}-H_{2m-2}CO-$,

n vaut 0 ou 1,

m vaut 2, 3 ou 4,

E représente S, SO ou $SO_2$ et

Ar représente un groupe phényle non substitué ou mono- ou disubstitué par alcoyle, O-alcoyle, S-alcoyle, SO-alcoyle, $SO_2$-alcoyle, OH, F, Cl, Br, $CF_3$ et/ou CN ou par un groupe méthylènedioxy et

où les groupes alcoyle possèdent chacun de 1 à 4 atomes de carbone,

ainsi que leurs sels d'addition d'acides physiologiquement acceptables.

2. 3-[4-(4-phényl-1,2,3,6-tétrahydropyridyl)-2--thiabutyl]-indole.

3. Procédé de préparation de dérivés d'indole contenant du soufre de formule générale I

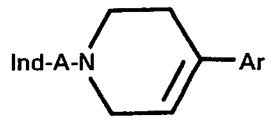

I

où

Ind représente un radical indol-3-yle, qui peut être mono- ou disubstitué par alcoyle, O-alcoyle, S-alcoyle, SO-alcoyle, $SO_2$-alcoyle, OH, F, Cl, Br, $CF_3$ et/ou CN par un groupe méthylènedioxy,

A représente $-(CH_2)_n-E-C_mH_{2m}-$ ou $-(CH_2)_n-E-C_{m-1}-H_{2m-2}-CO-$,

n vaut 0 ou 1,

m vaut 2, 3 ou 4,

E représente S, SO ou $SO_2$ et

Ar représente un groupe phényle non substitué ou mono- ou disubstitué par alcoyle, O-alcoyle, S-alcoyle, SO-alcoyle, $SO_2$-alcoyle, OH, F, Cl, Br, $CF_3$ et/ou CN ou par un groupe méthylènedioxy et

où les groupes alcoyle possèdent chacun de 1 à 4 atomes de carbone,

ainsi que leurs sels d'addition d'acides physiologiquement acceptables, caractérisé en ce qu'on fait réagir un compose de formule générale II

$$Ind-A-X^1 \qquad II$$

où

$X^1$ représente X ou $NH_2$ et

X représente Cl, Br, I, OH ou un groupe OH fonctionellement modifié réactif et

Ind et A ont les significations indiquées,

avec un composé de formule générale III

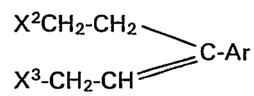

III

où

$X^2$ et $X^3$ peuvent être semplables ou différents et, si $X^1 = NH_2$, à chaque fois X, représentent autrement ensemble NH et

Ar a la signification indiquée,

ou en ce qu'on traite un composé correspondant sinon à la formule I, mais contenant cependant à la place d'un ou plusieurs atomes d'hydrogène un ou plusieurs groupe(s) réductible(s) et/ou une ou plusieurs liaisons C-C et/ou C-N supplémentaires, avec un agent réducteur,

ou en ce qu'on traite un composé correspondant sinon à la formule I, mais qui contient cependant à la place d'un ou plusieurs atomes d'hydrogène un ou plusieurs groupe(s) séparable(s) par solvolyse, avec un agent de solvolyse,

ou en ce qu'on fait réagir un composé de formule générale IV

$$Ind-CH_2N(R)_2 \qquad IV$$

où

R représente un alcoyle en C1 à C4, les deux radicaux R ensemble également $-(CH_2)_p-$ ou $-CH_2-CH_2OCH_2CH_2-$ et

p vaut 4 ou 5 et

Ind a la signification indiquée

avec un thiol de formule générale V

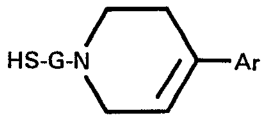

V

où

G représente $-C_mH_{2m}-$ ou $-C_{m-1}H_{2m-2}CO-$ et

m et Ar ont les significations indiquées ou un de ses sels,

ou en ce qu'on fait réagir un thiol de formule générale VI

Ind-SH VI

où

Ind a la signification indiquée

ou un de des sels

avec un composé de formule générale VII

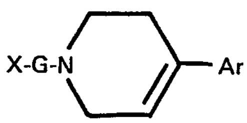

VII

où

X, G et Ar ont les significations indiquées

ou en ce qu'on traite un composé de formule VIII

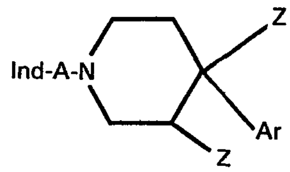

VIII

où

l'un des radicaux Z représente X, CN ou $NH_2$,

l'autre radical Z représente H et

Ind, A, Ar et X ont les significations indiquées,

avec un agent éliminant HZ,

et/ou en ce qu'éventuellement on réduit dans un composé de formule I un groupe CO en un groupe $CH_2$ et/ou en ce qu'on oxyde un groupe thioéther en un groupe SO ou $SO_2$ ou un groupe SO en un groupe $SO_2$ et/ou en ce qu'on clive un groupe alxoxy avec formation d'un groupe OH et/ou en ce qu'on transforme une base de formule I obtenue par traitement avec un acide en un sel d'addition d'acide physiologiquement acceptable.

4. Procédé de préparation de préparations pharmaceutiques, caractérisé en ce qu'on met sous une forme posologique appropriée un composé de formule I et/ou un de ses sels d'addition d'acides physiologiquement acceptables avec au moins un support ou additif solide, liquide ou semi-liquide et éventuellement en combinaison avec une ou plusieurs autres substances actives.

5. Préparation pharmaceutique caractérisée en ce qu'elle contient au moins un composé de formule générale I et/ou un de ses sels d'addition d'acides physiologiquement acceptables.

6. Composés de formule générale I selon la revendication 1 pour combattre les maladies.

7. Application de composés de formule générale I et/ou de leurs sels d'addition d'acides physiologiquement acceptables à la préparation de médicaments.